# EUROPEAN PATENT APPLICATION

(11) **EP 3 831 542 A1**
(43) Date of publication of application: **09.06.2021**
(21) Application number: 20164600.7
(22) Date of filing: 20.03.2020
(51) Int. Cl.: B25J 9/04, B25J 19/02, B25J 9/00, B25J 9/02, A01D 46/30

(54) **LEAVES SPREADING MECHANISM AND PLANT RECOGNITION SYSTEM**

(30) Priority: 06.12.2019 TW 108144754
(71) Applicant: Industrial Technology Research Institute, 31040 Hsinchu (TW)
(72) Inventor: Lin, Keng-Hsun, 31040 Chutung, Hsinchu (TW)
(74) Representative: Papula Oy

(57) **Abstract**

A leaves spreading mechanism is provided, including a leaves spreading device and a first driver. The first driver drives the leaves spreading device to move left and right in a first direction and spread leaves of a plant at a position-to-be-tested. A plant recognition system including the leaves spreading mechanism is also provided.

## Description

### TECHNICAL FIELD

The present disclosure relates to a leaves spreading mechanism and a plant recognition system including the leaves spreading mechanism.

### BACKGROUND

Currently, plants are graded through manpower, who can neither guarantee the quality of the grading nor identify defects of the leaves. In order to identify the defects of the leaves, the leaves have to be cut and then identified by a measurement device. Doing so will damage the plants.

Therefore, how to solve the problem of the prior art is becoming an urgent issue in the art.

### SUMMARY

Provided is a leaves spreading mechanism (100, 200) according to the present disclosure, comprising: a leaves spreading device (1, 1'); and a first driver (2) configured for driving the leaves spreading device (1, 1') to move left and right in a first direction (X) to spread leaves of a plant (4) at a position-to-be-tested.

Also provided is a plant recognition system (300) according to the present disclosure, comprising: a leaves spreading mechanism (100, 200) comprising: a leaves spreading device (1, 1') including a leaves spreading frame (11, 11') and at least one attachment (12) disposed on the leaves spreading frame (11, 11'); and a first driver (2) configured for driving the leaves spreading device (1, 1') to move left and right in a first direction (X) to enable the leaves spreading frame (11, 11') of the leaves spreading device (1, 1') to spread leaves of a plant (4) at a position-to-be-tested; and an image processing module (72) configured for controlling the attachment (12) to capture an image of the spread leaves and identify features of the leaves.

### BRIEF DESCRIPTION OF DRAWINGS

FIG. 1 schematically illustrates a leaves spreading mechanism of a first embodiment according to the present disclosure;
FIG. 2 schematically illustrates a leaves spreading device of a leaves spreading mechanism of a first embodiment according to the present disclosure;
FIG. 3 schematically illustrates a leaves spreading frame of a leaves spreading device of a leaves spreading mechanism of a first embodiment according to the present disclosure;
FIG. 4 explosively illustrates a leaves spreading device of a leaves spreading mechanism of a first embodiment according to the present disclosure;
FIG. 5 schematically illustrates a leaves spreading mechanism spreading leaves of a plant of a first embodiment according to the present disclosure;
FIG. 6 schematically illustrates a servo motor of a leaves spreading mechanism of a first embodiment according to the present disclosure;
FIG. 7 schematically illustrates a leaves spreading mechanism of a second embodiment according to the present disclosure;
FIG. 8 schematically illustrates a plant recognition system according to the present disclosure;
FIG. 9 schematically illustrates a plant according to the present disclosure;
FIG. 10 is a functional block diagram of a microprocessor according to the present disclosure; and
FIGs. 11 to 13 schematically illustrate a plant recognition system according to the present disclosure.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

It should be appreciated that the structures, proportions, size and the like of the figures in the present application are intended to be used in conjunction with the disclosure of the specification. They are not intended to limit the present disclosure and therefore do not represent any substantial technical meanings. The details of the specification may be on the basis of different points and applications, and numerous modifications and variations can be devised without departing from the spirit of the present disclosure. As used herein, the terms "first," "second," "a" and the like, are used to distinguish one element from another, and are not intended to limit the scope of the present application. Changes or adjustments are considered to be within the scope of the present disclosure, without departing from the scope of the present disclosure.

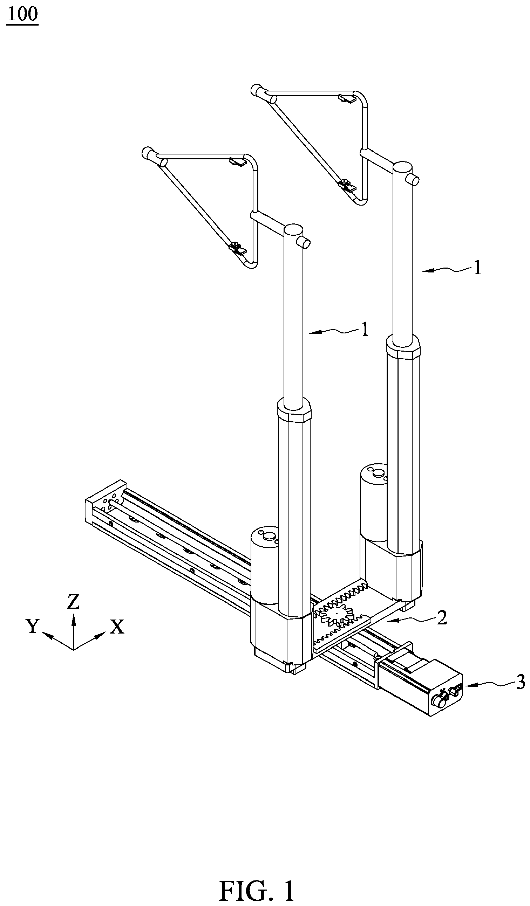

FIG. 1 schematically illustrates a leaves spreading mechanism 100 of a first embodiment according to the present disclosure. The leaves spreading mechanism 100 comprises a first driver 2, two leaves spreading devices 1 disposed on two opposing sides of the first driver 2, respectively, and a second driver 3. In an embodiment, the leaves spreading mechanism 100 includes any number of the leaves spreading devices 1. In an embodiment, the leaves spreading device(s) 1 is disposed at any portion of the first driver 2, e.g., a center.

In an embodiment, the two leaves spreading devices 1 are disposed on two opposing sides of the first driver 2 respectively and driven by the first driver 2 to move left and right in a first direction (e.g., X-axis in FIG. 1). The first driver 2 is disposed on the second driver 3, and the second driver 3 drives the first driver 2 and the leaves spreading device 1 to move back and forth in a second direction (e.g., Y-axis in FIG. 1).

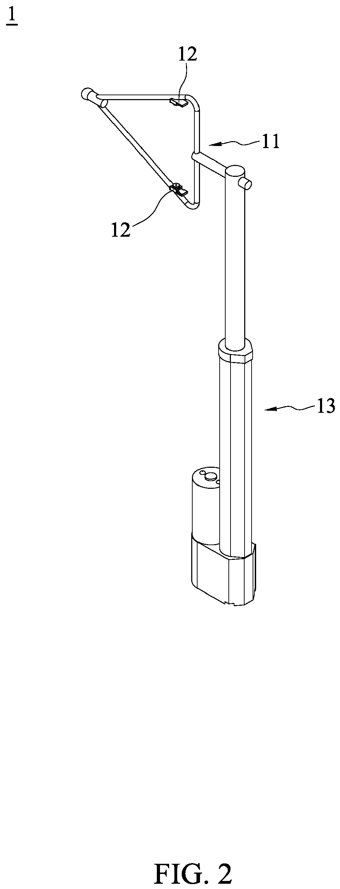

FIG. 2 schematically illustrates a leaves spreading device 1 of a first embodiment according to the present disclosure. The leaves spreading device 1 includes a leaves spreading frame 11, two attachments 12 and a third driver 13. The third driver 13 is connected to the leaves spreading frame 11 and the first driver 2 (e.g., one of the two opposing sides of the first driver 2). The attachments 12 ae disposed on the leaves spreading frame 11. The third driver 13 drives the leaves spreading frame 11 to move up and down in a third direction (e.g., Z-axis in FIG. 1). In an embodiment, any number of the attachments 12 are disposed on the leaves spreading frame 11. In an embodiment, the attachments 12 include at least one of a micro camera, a liquid sprayer and a micro video camera or a combination thereof. The micro camera and the micro video camera capture an image of leaves of a plant. The liquid sprayer sprays liquid on the leaves.

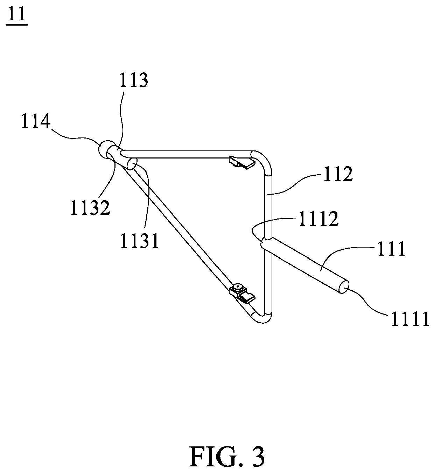

FIG. 3 schematically illustrates a leaves spreading frame 11 of a first embodiment according to the present disclosure. The leaves spreading frame 11 includes a first connector 111, a triangular member 112, a second connector 113 and a ball-shaped member 114 that are integrated with one another or manufactured individually.

The first connector 111 has a first end 1111 connected to the third driver 13, and a second end 1112 opposing the first end 1111 and connected to the triangular member 112.

The second connector 113 has a first end 1131 connected to the triangular member 112, and a second end 1132 opposing the first end 1131 and connected to the ball-shaped member 114.

The triangular member 112 is constituted by three sides, one of which is connected to the second end 1112 of the first connector 111, and the others of which form an angle connected to the second connector 113.

The three sides of the triangular member 112 are integrated with one another or manufactured individually. In an embodiment, a smooth curve is formed at an intersection of two of the three sides of the triangular member 112 that are not connected to the first connector 111, with the second connector 113 and the ball-shaped member 114 omitted.

In an embodiment, the two attachments 12 are disposed on the two of the three sides of the triangular member 112 that are not connected to the first connector 111. In an embodiment, the two attachments 12 are disposed on any one or two of the sides of the triangular member 112.

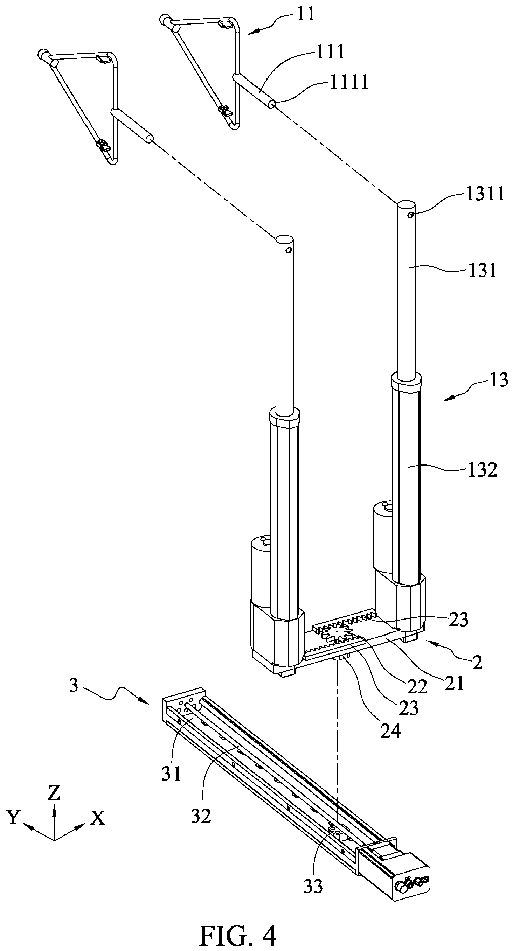

FIG. 4 explosively illustrates the leaves spreading device 1 according to the present disclosure. The first driver 2 includes a board 21, gears 22, a pair of gear racks 23, and a coupling stand 24. The gears 22 and the pair of gear racks 23 are disposed on a first surface (i.e., an upper surface) of the board 21. The coupling stand 24 is disposed on a second surface (i.e., a lower surface) of the board 21. The pair of gear racks 23 are engaged with the gears 22 and a first transmission member 132 of the third driver 13 of the leaves spreading device 1, respectively. The gears 22 drive the pair of gear racks 23 and the leaves spreading device 1 to move left and right in a first direction (e.g., X-axis in FIG. 4).

The third driver 13 comprises a lifting rod 131 and a first transmission member 132. The first transmission member 132 drives the lifting rod 131 to move up and down. The lifting rod 131 has a through hole 1311. The manner in which the first connector 111 of the leaves spreading frame 11 connects to the third driver 13 is that the first end 1111 of the first connector 111 passes through the through hole 1311. In an embodiment, the through hole 1311 is formed on a side of the lifting rod 131 opposing the first transmission member 132.

The second driver 3 includes a slide groove 31, a second transmission member 33 and a slide rail 32. The second transmission member 33 is disposed in the slide groove 31. The slide rail 32 is disposed above the second transmission member 33 in the slide groove 31. The second transmission member 33 is coupled to the coupling stand 24 of the first driver 2. The second transmission member 33 drives the coupling stand 24 of the first driver 2 to move on the slide rail 32.

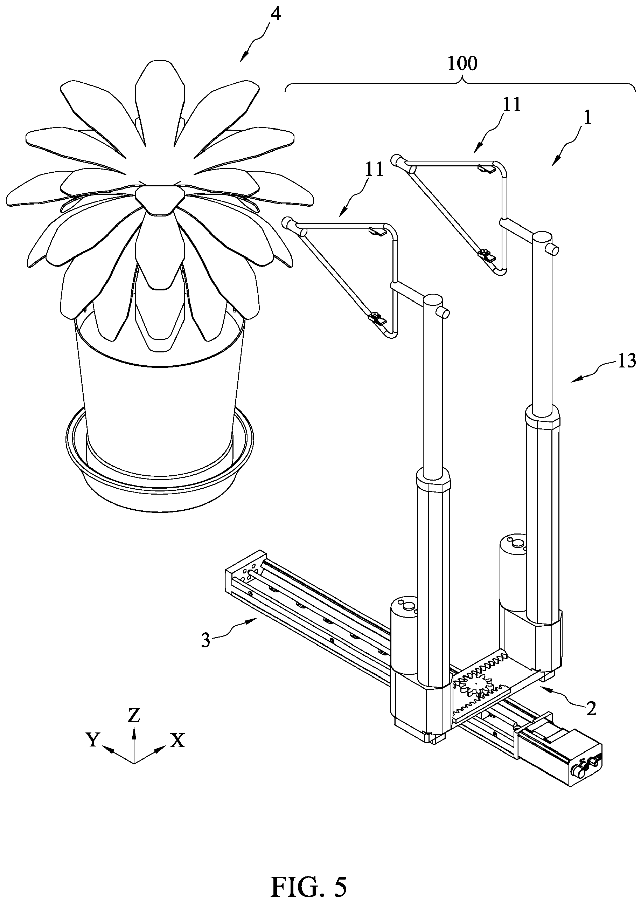

FIG. 5 schematically illustrates the leaves spreading mechanism 100 spreading leaves of a plant of a first embodiment according to the present disclosure. The third driver 13 of the leaves spreading device 1 drives the leaves spreading frame 11 to move up and down in a third direction (e.g., Z-axis in FIG. 5), to adjust the leaves spreading frame 11 to be leveled with the leaves of the plant 4. The second driver 3 drives the leaves spreading device 1 to move in Y-axis toward the plant 4 at a position-to-be-tested, to drive the leaves spreading frame 11 of the leaves spreading device 1 to be inserted among the leaves of the plant 4. After the leaves spreading frame 11 of the leaves spreading device 1 is inserted among the leaves of the plant 4, the first driver 2 drives the leaves spreading device 1 to move left and right in a first direction, to drive the leaves spreading frame 11 of the leaves spreading device 1 to spread the leaves of the plant 4 at the position-to-be-tested. In an embodiment, the attachment 12 is a micro camera or a micro video camera, and captures an image of the spread leaves. In another embodiment, the attachment 12 is a liquid sprayer, and sprays liquid on the faces of the spread leaves. The position-to-be-tested is located right in front of the leaves spreading mechanism 100. In an embodiment, each of the third drivers 13 drives the leaves spreading frame 11 to be leveled with the leaves of the plant 4. In another embodiment, each of the third drivers 13 drives the leaves spreading frame 11 not to be leveled with the leaves of the plant 4 (i.e., each of the lifting rods 131 of the third drivers 13 can be at different heights).

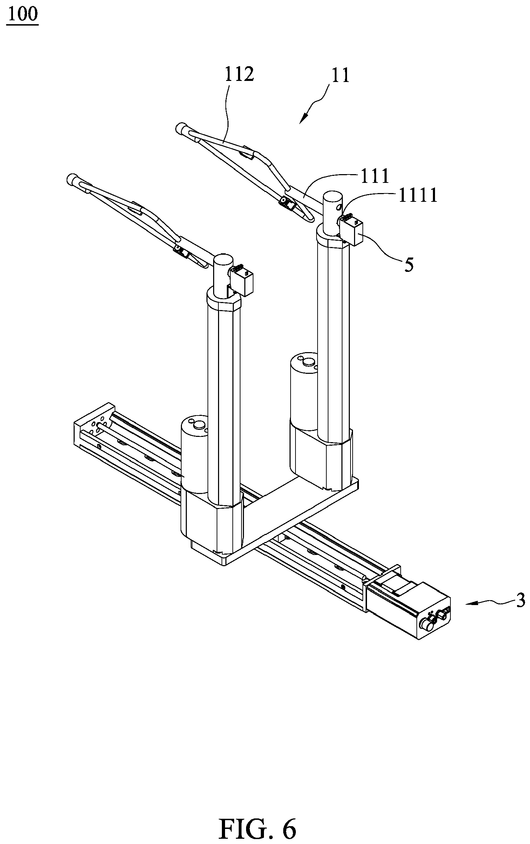

FIG. 6 schematically illustrates a servo motor of a leaves spreading mechanism of a first embodiment according to the present disclosure. In an embodiment, a servo motor 5 is disposed on the first end 1111 of the first connector 111 of the leaves spreading frame 11, and drives the first connector 111 to rotate to drive the triangular member 112 to rotate. The triangular member 112 is adjusted by rotating itself to be inserted into a suitable position among the leaves of the plant 4. The second driver 3 drives the leaves spreading frame 11 to be inserted among the leaves of the plant 4. In an embodiment, when the leaves spreading frame 11 is among the leaves of the plant 4, the servo motor 5 drives the triangular member 112 to rotate and spread the leaves.

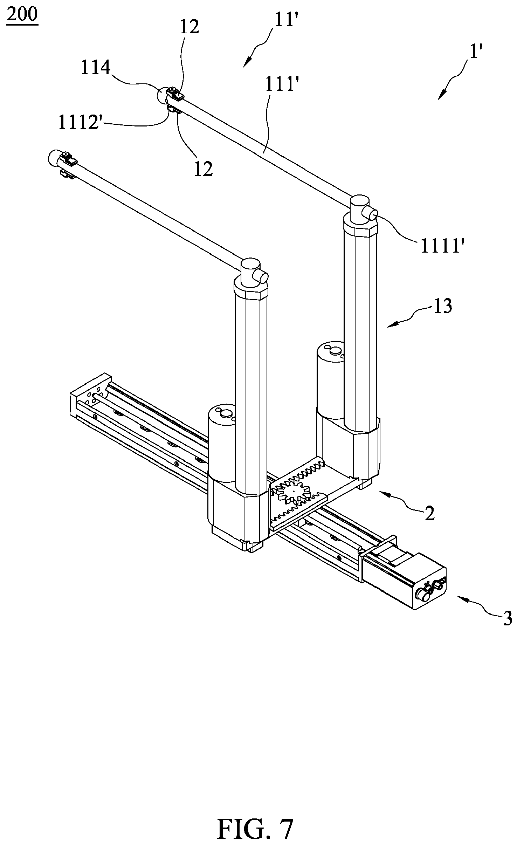

FIG. 7 schematically illustrates a leaves spreading mechanism 200 of a second embodiment according to the present disclosure. The second embodiment differs from the first embodiment in that the triangular member 112 and the second connector 113 of the leaves spreading frame 11 are omitted in the leaves spreading device 1', the attachment 12 is disposed at a different location, and the length of the first connector 111 is changed.

In the leaves spreading device 1' of the second embodiment, the leaves spreading frame 11' comprises a first connector 111' and a ball-shaped member 114. The first connector 111' has a first end 1111' and a second end 1112' opposing the first end 1111'. The first end 1111' and the third driver 13 are connected in the same manner as the first end 1111 and the third driver 13 of the leaves spreading mechanism 100 do in the first embodiment. The first end 1111' passes through a through hole in the lifting rod 131 of the third driver 13. The second end 1112' is connected to the ball-shaped member 114. The leaves spreading frame 11' is or is not integrally formed. In an embodiment, the first connector 111' is longer than the first connector 111.

In an embodiment, two attachments 12 are disposed on a side of the first connector 111' away from the third driver 13. In another embodiment, any number of the attachment 12 is disposed.

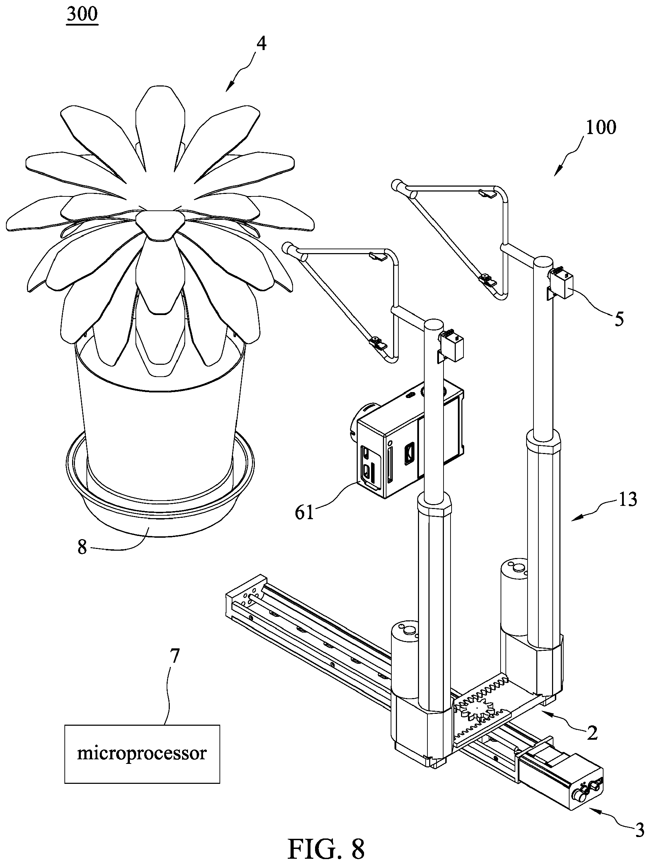

A plant recognition system is also provided. The plant recognition system employs the leaves spreading mechanism 100 of FIG. 1 or the leaves spreading mechanism 200 of FIG. 7. In an embodiment, the attachment 12 of the plant recognition system is one of a micro camera and a micro video camera or a combination thereof, and captures an image of the leaves of the plant 4. The schematic diagram of a plant recognition system 300 of FIG. 8 is described based on the leaves spreading mechanism 100 of FIG. 1.

As shown in FIG. 8, the plant recognition system 300 comprises a leaves spreading mechanism 100, a first image capturing device 61, a microprocessor 7 and a turning plate 8. The microprocessor 7 is connected to the first image capturing device 61, the turning plate 8 and the leaves spreading mechanism 100 in a wired or wireless manner.

The turning plate 8 carries and turning the plant 4.

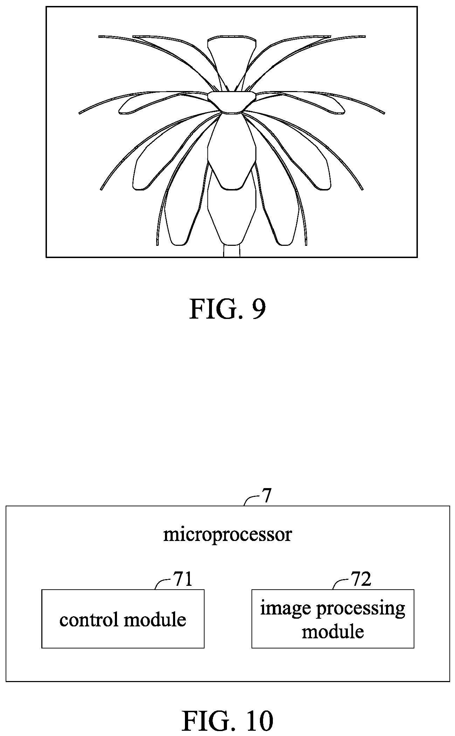

The first image capturing device 61 is disposed at the position-to-be-tested in front of the plant 4 and captures a first image of a front face of the plant 4 (as shown in FIG. 9). The front face of the plant 4 faces the leaves spreading mechanism 100.

In an embodiment, the first image capturing device 61 is disposed on the third driver 13.

FIG. 10 schematically illustrates a microprocessor 7 according to the present disclosure. The microprocessor 7 comprises a control module 71 and an image processing module 72. In an embodiment, the control module 71 and the image processing module 72 are software executable by the microprocessor 7.

The control module 71 receives the first image captured by the first image capturing device 61, and controls the operations of the first driver 2, the second driver 3, the third driver 13, the turning plate 8 and/or the servo motor 5 based on the first image captured by the first image capturing device 61.

The image processing module 72 receives images of leaves of the plant 4 captured by the micro camera, and identifies features of the leaves based on the images. In an embodiment, the features of the leaves include the defects, color, length and width of the leaves.

The control module 71, after receiving the first image captured by the first image capturing device 61, determines the position of the leaves of the plant 4 based on the first image, and controls the third driver 13 to adjust the leaves spreading frame 11 to be flush with the leaves based on the determined position of the leaves. In an embodiment, different leaves spreading frames 11 are adjusted to be at the same height. In another embodiment, the lifting rods 131 driven by different third drivers 13 are controlled based on the position of the leaves to adjust different leaves spreading frame 11 to be at different height.

After determining from the first image that the leaves spreading frame 11 cannot be inserted among the leaves of the plant 4, the control module 71 controls the servo motor 5 to drive the triangular member 112 to rotate such that the leaves spreading frame 11 rotates and is adjusted to a suitable position, at which the leaves spreading frame 11 is allowed to be inserted among the leaves of the plant 4. In another embodiment, the control module 71 controls the turning plate 8 to rotate to adjust the leaves of the plant 4 disposed on the turning plate 8 to a suitable position, at which the leaves spreading frame 11 is allowed to be inserted among the leaves of the plant 4.

After determining from the first image that the leaves spreading frame 11 can be inserted among the leaves of the plant 4, the control module 71 controls the second driver 3 to drive the leaves spreading frame 11 to be inserted among the leaves of the plant 4.

After the leaves spreading frame 11 is inserted among the leaves of the plant 4, the control module 71 controls the first driver 2 or the servo motor 5 to drive the leaves spreading frame 11 to spread the leaves of the plant 4.

When the leaves spreading frame 11 spreads the leaves of the plant 4, the image processing module 72 controls the micro camera to capture the image of the leaves of the plant 4, receives the image of the leaves captured by the micro camera, and identifies the features of the leaves based on the image of the leaves. In an embodiment, the image processing module 72 controls the micro camera to capture the images of front and rear sides of the same leaves or the images of different leaves.

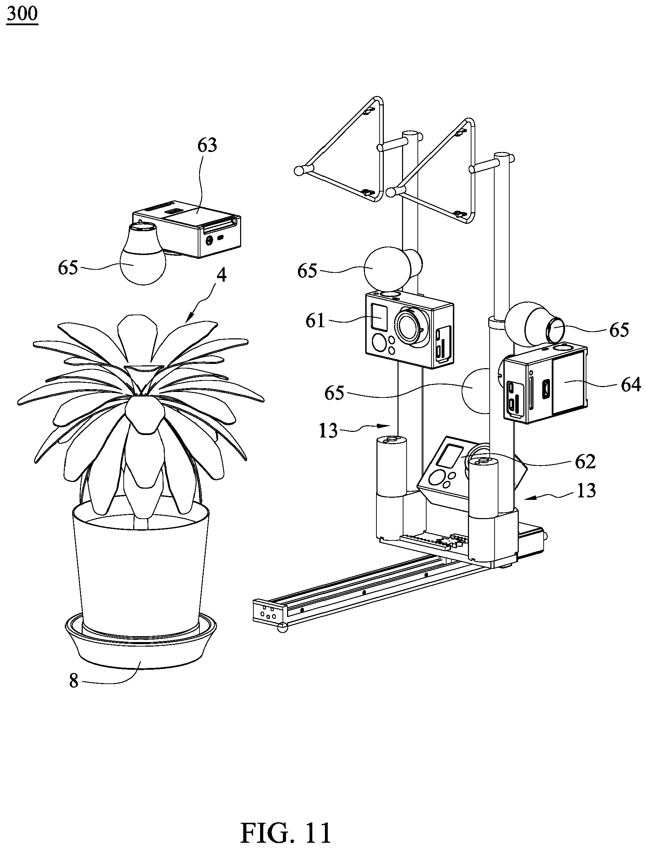

FIGs. 11-13 schematically illustrate a plant recognition system according to the present disclosure. As shown in FIG. 11, the plant recognition system 300 further comprises a second image capturing device 62, a third image capturing device 63, a fourth image capturing device 64, and a light source 65 disposed by the first image capturing device 61, the second image capturing device 62, the third image capturing device 63 and the fourth image capturing device 64. In an embodiment, the light source 65 projects light onto the plant 4.

The second image capturing device 62 is disposed 45 degrees below in front of the plant 4 disposed on the position-to-be-tested and on the third driver 13. The third image capturing device 63 is disposed above the plant 4 disposed on the position-to-be-tested. The fourth image capturing device 64 is disposed on the right or left side of the plant 4 disposed on the position-to-be-tested. The second image capturing device 62, the third image capturing device 63 and the fourth image capturing device 64 may provide second to fourth images of the plant 4 disposed on the position-to-be-tested to the control module 71 of the microprocessor 7, for the control module 71 to determine the position of the plant 4 or the position of the leaves of the plant 4.

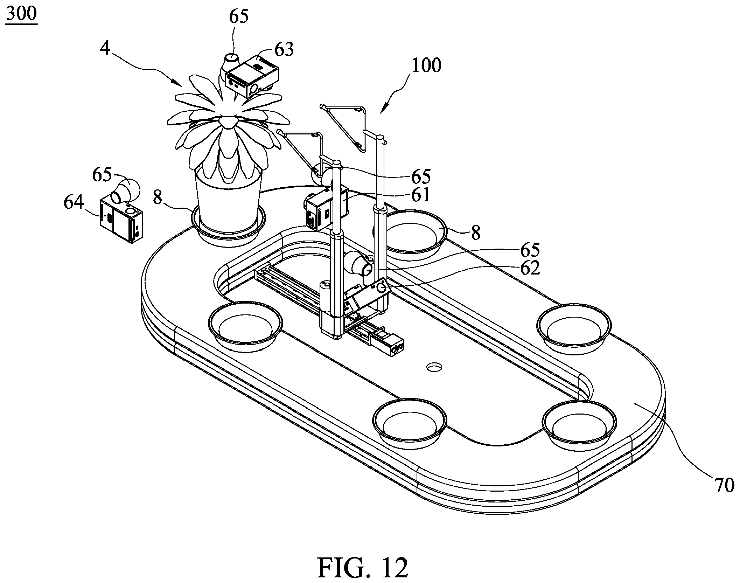

As shown in FIG. 12, the plant recognition system 300 further comprises a conveyance device 70. In an embodiment, the conveyance device 70 is a conveyer belt that surrounds the leaves spreading mechanism 100. The turning plate 8 is disposed on the conveyance device 70. The conveyance device 70 conveys the plant 4 on the turning plate 8 to the position-to-be-tested right in front of the leaves spreading mechanism 100. The microprocessor 7 controls, based on the first image captured by the first image capturing device 61 or the first to fourth images captured by the first to fourth image capturing devices 61 to 64, respectively, the leaves spreading mechanism 100 and the turning plate 8 to spread the leaves of the plant 4, capture the images of the spread leaves, and identify the features of the leaves.

In another embodiment, the conveyance device 70 is a manipulator that conveys the plant 4 to the position-to-be-tested right in front of the leaves spreading mechanism 100. In an embodiment, the turning plate 8 is disposed at the bottom of the plant 4, and is conveyed, together with the plant 4, by the manipulator. In another embodiment, the turning plate 8 is disposed at the position-to-be-tested, and the manipulator conveys the plant 4 to the turning plate 8 at the position-to-be-tested.

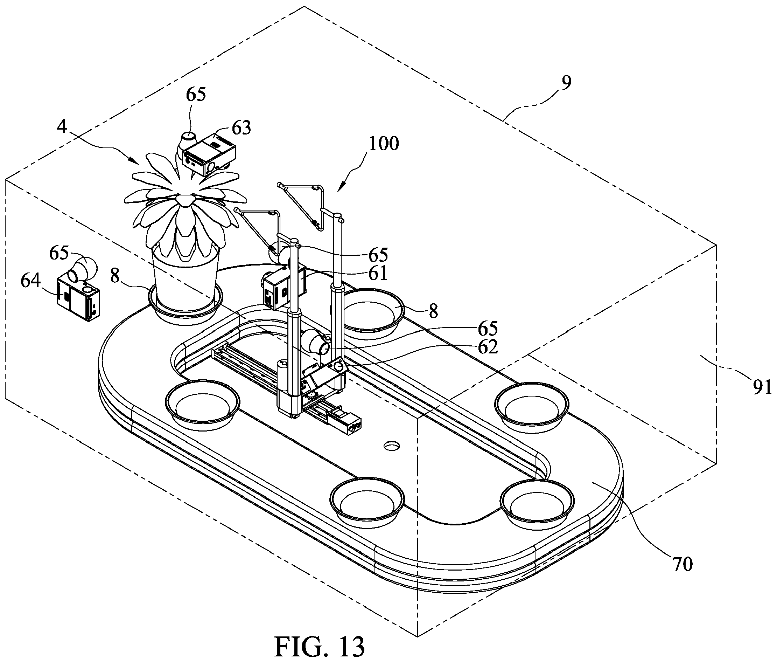

As shown in FIG. 13, the plant recognition system 300 is disposed in a housing 9. The housing 9 has an opening 91. The opening 91 provides another manipulator to convey the plant 4 to the turning plate 8 on the conveyance device 70. The conveyance device 70 conveys the plant 4 to the position-to-be-tested right in front of the leaves spreading mechanism 100. The microprocessor 7 controls, based on the first image captured by the first image capturing device 61 or the first to fourth images captured by the first to fourth image capturing devices 61 to 64, respectively, the leaves spreading mechanism 100 and the turning plate 8 to spread the leaves, capture the images of the spread leaves, and identify the features of the leaves.

It is thus known from the above that in the leaves spreading mechanism and the plant recognition system according to the present disclosure, the first to third drivers, the servo motor and the turning plate allow the leaves spreading frame to easily spread leaves at any angle, and the image processing module can identify features of an image of the spread leaves captured by a micro camera disposed on the leaves spreading frame. Therefore, the identifying of the quality of the plants is improved.

It will be apparent to those skilled in the art that various modifications and variations can be made to the disclosed embodiments. It is intended that the specification and examples be considered as exemplary only, with a true scope of the present disclosure being indicated by the following claims and their equivalents.

## Claims

1. A leaves spreading mechanism (100, 200), comprising:
a leaves spreading device (1, 1'); and
a first driver (2) configured for driving the leaves spreading device (1, 1') to move left and right in a first direction (X) to spread leaves of a plant (4) at a position-to-be-tested.

2. The leaves spreading mechanism (100, 200) of claim 1, wherein the leaves spreading device (1, 1') comprises a leaves spreading frame (11, 11') and at least one attachment (12) disposed on the leaves spreading frame (11, 11').

3. The leaves spreading mechanism (100, 200) of claim 2, wherein the attachment (12) is one of a micro camera, a liquid sprayer and a micro video camera or a combination thereof.

4. The leaves spreading mechanism (100, 200) of claim 2, wherein the leaves spreading frame (11, 11') comprises a first connector (111, 111') and a second connector (113), each of which has a first end (1111, 1111', 1131) and a second end (1112, 1112', 1132) opposing the first end (1111, 1111', 1131), wherein the leaves spreading frame (11, 11') further comprises a ball-shaped member (114) connected to the second end (1132) of the second connector (113), wherein the leaves spreading frame (11, 11') further comprises a triangular member (112) constituted by three sides, one of which is connected to the second end (1112, 1112') of the first connector (111, 111'), wherein a smooth curve is formed at an intersection of two of the three sides that are not connected to the first connector (111, 111'), or the ball-shaped member (114) connected to the intersection, wherein the leaves spreading device (1, 1') further comprises a servo motor (5) disposed on the first end (1111, 1111') of the first connector (111, 111') of the leaves spreading frame (11, 11') and configured for rotating the first connector (111, 111') of the leaves spreading frame (11, 11'), and wherein the leaves spreading device (1, 1') further comprises a third driver (13) connected to the first driver (2) and the first end (1111, 1111') of the first connector (111, 111') of the leaves spreading frame (11, 11') and configured for driving the leaves spreading frame (11, 11') to move up and down in a third direction (Z).

5. The leaves spreading mechanism (100, 200) of claim 2, wherein the leaves spreading frame (11, 11') comprises:
a first connector (111, 111') having a first end (1111, 1111') and a second end (1112, 1112') opposing the first end (1111, 1111'); and
a ball-shaped member (114) connected to the second end (1112, 1112') of the first connector (111, 111').

6. The leaves spreading mechanism (100, 200) of claim 1, further comprising a second driver (3) connected to the first driver (2) and configured for driving the first driver (2) to move back and forth in a second direction (Y) to drive the leaves spreading device (1, 1') to be inserted among the leaves of the plant (4) disposed at the position-to-be-tested.

7. A plant recognition system (300), comprising:
a leaves spreading mechanism (100, 200) comprising:
a leaves spreading device (1, 1') including a leaves spreading frame (11, 11') and at least one attachment (12) disposed on the leaves spreading frame (11, 11'); and
a first driver (2) configured for driving the leaves spreading device (1, 1') to move left and right in a first direction (X) to enable the leaves spreading frame (11, 11') of the leaves spreading device (1, 1') to spread leaves of a plant (4) at a position-to-be-tested; and
an image processing module (72) configured for controlling the attachment (12) to capture an image of the spread leaves and identify features of the leaves.

8. The plant recognition system (300) of claim 7, further comprising a conveyance device (70) configured for conveying the plant (4) to the position-to-be-tested.

9. The plant recognition system (300) of claim 8, wherein the conveyance device (70) is a conveyer belt or a manipulator.

10. The plant recognition system (300) of claim 7, further comprising a first image capturing device (61) configured for capturing a first image of the plant (4) at the position-to-be-tested facing a front face of the leaves spreading mechanism (100, 200).

11. The plant recognition system (300) of claim 10, further comprising a turning plate (8) configured for carrying and turning the plant (4), and a control module (71) configured for controlling the turning plate (8) to turn the plant (4) based on the first image.

12. The plant recognition system (300) of claim 7, wherein the leaves spreading mechanism (100, 200) further comprises a second driver (3) connected to the first driver (2) and configured for driving the first driver (2) to move back and forth in a second direction (Y) to drive the leaves spreading device (1, 1') to be inserted among the leaves of the plant (4) disposed at the position-to-be-tested.

13. The plant recognition system (300) of claim 7, wherein the leaves spreading frame (11, 11') further comprises a first connector (111, 111') and a second connector (113), each of which has a first end (1111, 1111', 1131) and a second end (1112, 1112', 1132) opposing the first end (1111, 1111', 1131), wherein a ball-shaped member (114) is connected to the second end (1132) of the second connector (113), wherein the leaves spreading frame (11, 11') further comprises a triangular member (112) constituted by three sides, one of which is connected to the second end (1112, 1112') of the first connector (111, 111'), wherein a smooth curve is formed at an intersection of two of the three sides that are not connected to the first connector (111, 111'), or the ball-shaped member (114) connected to the intersection, wherein the leaves spreading device (1, 1') further comprises a servo motor (5) disposed on the first end (1111, 1111') of the first connector (111, 111') and configured for driving the first connector (111, 111') to rotate, and wherein the leaves spreading device (1, 1') further comprises a third driver (13) connected to the first driver (2) and the first end (1111, 1111') of the first connector (111, 111') of the leaves spreading frame (11, 11') and configured for driving the leaves spreading frame (11, 11') to move up and down in a third direction (Z).

14. The plant recognition system (300) of claim 7, wherein the leaves spreading frame (11, 11') further comprises:
a first connector (111, 111') having a first end (1111, 1111') and a second end (1112, 1112') opposing the first end (1111, 1111'); and
a ball-shaped member (114) connected to the second end (1112, 1112') of the first connector (111, 111').

15. The plant recognition system (300) of claim 7, wherein the attachment (12) is one of a micro camera and a micro video camera or a combination thereof.
